(19) Europäisches Patentamt
European Patent Office
Office européen des brevets

(11) EP 0 843 993 B1

(12) EUROPÄISCHE PATENTSCHRIFT

(45) Veröffentlichungstag und Bekanntmachung des Hinweises auf die Patenterteilung:
**06.08.2003 Patentblatt 2003/32**

(51) Int Cl.[7]: **A61K 7/13**

(21) Anmeldenummer: **97117200.2**

(22) Anmeldetag: **04.10.1997**

# (54) Mittel und Verfahren zum Färben keratinischer Fasern

Composition and process for dyeing hair

Composition et procédé pour colorer les cheveux

(84) Benannte Vertragsstaaten:
**DE ES FR GB IT**

(30) Priorität: **20.11.1996 DE 19648019**

(43) Veröffentlichungstag der Anmeldung:
**27.05.1998 Patentblatt 1998/22**

(73) Patentinhaber: **Wella Aktiengesellschaft
64274 Darmstadt (DE)**

(72) Erfinder:
- **Semadeni, Pascal André, Dr.
1792 Cordast (CH)**
- **Braun, Hans-Jürgen, Dr.
3182 Überstorf (CH)**

(56) Entgegenhaltungen:
**US-A- 5 364 767**

- **PATENT ABSTRACTS OF JAPAN vol. 017, no. 324 (M-1433), 21. Juni 1993 (1993-06-21) & JP 05 038875 A (JUJO PAPER CO LTD), 19. Februar 1993 (1993-02-19)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 843 993 B1

Printed by Jouve, 75001 PARIS (FR)

## Beschreibung

Die vorliegende Erfindung betrifft ein Mittel sowie ein Verfahren zur Färbung von keratinischen Fasern, insbesondere menschlichen Haaren.

Die Verwendung von gemahlenen Pflanzen zur Färbung von Haaren ist im Prinzip bereits aus dem Altertum bekannt. Üblicherweise werden hierzu gepulverte Pflanzenteile von stark färbenden Pflanzen, wie zum Beispiel Indigo-Blätter oder Henna verwendet, welche mit Wasser, Füllstoffen und Verdickungsmitteln zu einer Färbepaste angerührt werden und sodann auf das Haar aufgetragen werden. Diese Verfahrensweise besitzt jedoch den Nachteil, daß die Farbstoffpulver sehr fein vermahlen sein müssen, um auf dem Haar eine gleichmäßige Färbung zu erzeugen. Außerdem dringt in vielen Fällen der Farbstoff nicht in das Haar ein und es verbleiben wasserunlösliche Bestandteile auf dem Haar, die nur sehr schwer vollständig auswaschbar sind. Eine feine Vermahlung der Farbstoffe hat zudem den Nachteil, daß die Herstellung und Verarbeitung mit einer unangenehmen Staubentwicklung verbunden ist. Es wurde daher bereits versucht, Naturfarbstoffe aus den entsprechenden Pflanzen zu extrahieren und die Extrakte in Färbezubereitungen einzuarbeiten. Die durch Extraktion aus Pflanzen isolierten Farbstoffe sind jedoch gegenüber den Einflüssen von Sauerstoff und Licht sehr instabil und eignen sich daher nicht zur Herstellung von lagerfähigen Rezepturen.

Die Indigofera-Pflanzen enthalten Indican (Farbvorstufe des Indigos), welches durch Hydrolyse leicht in Indoxyl und Glucose gespalten werden kann. Das hierbei erhaltene Indoxyl wird bereits bei der Einwirkung von Luftsauerstoff in Indigo überführt. Indigo ist ein blauer Farbstoff mit guten Echtheitseigenschaften und dient seit Jahrtausenden zum Färben von Textilmaterialien. Da Indigo wasserunlöslich ist, muß dieser Farbstoff zum Färben über einen Reduktionsprozess, beispielsweise mittels einer alkalischen Hydrogensulfitlösung, in eine wasserlösliche Form (die sogenannte Indigoküpe) überführt werden werden. Eine derartige Vorgehensweise ist jedoch für Haarfärbungen wenig praktikabel.

US-A-5 364 767 offenbart chromogene Verbindungen der Formel I. Die verwendung von 5-Bromo-4-chloro-3-indolyl-β-O-galactopyranoside als ein Indikator wird in US-A-5 364 767 besprochen.

Es wurde nunmehr überraschenderweise gefunden, daß durch die Verwendung einer geeigneten Indigovorstufe gemäß der allgemeinen Formel (I) in Gegenwart von Alkalien blaue Haarfärbungen erhalten werden, die die vorgenannten Nachteile nicht aufweisen.

Gegenstand der vorliegenden Erfindung ist daher ein Mittel zur Färbung von Haaren, das unmittelbar vor der Anwendung durch Vermischen (i) einer Farbträgermasse, enthaltend eine Verbindung der allgemeinen Formel (I)

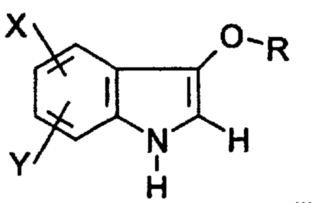

(I) ,

in der R gleich einer DL-, D- oder L-Aldopyranose beziehungsweise DL-, D- oder L-Ketopyranose ist und die Reste X und Y unabhängig voneinander Wasserstoff, ein Halogenatom (insbesondere Chlor, Brom oder Jod), eine Hydroxygruppe oder eine Alkoxygruppe mit 1 bis 4 Kohlenstoffatomen bedeuten, mit (ii) einer Hydrolase erhalten wird.

Bevorzugte Verbindungen gemäß der allgemeinen Formel (I) sind die 5-Brom-4-chlor-3-indolyl-β-D-galactoside.

Zur Optimierung des Farbergebnisses sowie zur Erzeugung von speziellen Farbeffekten können dem Haarfärbemittel weiterhin Verbindungen aus der Gruppe der direktziehenden Farbstoffe, beispielsweise aromatische Nitrofarbstoffe, Azofarbstoffe, Anthrachinon-Farbstoffe oder Triphenylmethan-Farbstoffe, alleine oder im Gemisch miteinander zugesetzt werden.

Beispiele für Nitrofarbstoffe sind Pikraminsäure, 4-(2',3'-Dihydroxypropyl)-amino-3-nitro-trifluor-methylbenzol, 4,N-Ethyl-N-(2'-hydroxyethyl)-amino-1-(2'-hydroxyethyl)-amino-2-nitro-benzol, 2-Chlor-6-ethylamino-4-nitrophenol, 1-Hydroxy-2-ß-hydroxyethylamino-4,6-dinitrobenzol, 4-(2'-Hydroxyethyl)-amino-3-nitro-chlorbenzol, 2-Amin-6-chlor-4-nitrophenol und 4-(2'-Hydroxyethyl)-amino-3-nitro-methylbenzol.

Beispiele für Azofarbstoffe sind 1-(2'-Methoxyphenylazo)-2-hydroxy-7-trimethyl-ammonium-naphthalin (Basic Red 76), 4-(4'-Sulfo-1-phenylazo)-1-(4"-sulfophenyl)-3-carboxy-5-hydroxypyrazolon (Acid Yellow 23), 4-Amino-4'-Bis[2"-hydroxyethyl]-amino-azobenzol (Disperse Black 9) und 1-(4'-Aminophenylazo)-2-hydroxy-7-trimethyl-ammonium-naphthalin (Basic Braun 16).

Beispiele für Anthrachinon-Farbstoffe sind 1-Methylamino-4-(2'hydroxyethyl)amino-anthrachinon (Disperse Blue 3), 1-Amino-4-hydroxyanthrachinon (Disperse Red 15), 2-Methoxy-1,4-diamino-anthrachinon (Disperse Red 11),

1,4-Diamino-anthrachinon (Disperse Violet 1), 1-Amino-4-methylamino-anthrachinon (Disperse Violet 4), 1,4-Bis(2',3'-Dihydroxypropyl)amino-anthrachinon, 1-Methylamino-4-(amino-npropyltrimethyl-ammonium)-anthrachinon methylsulfat (Basic Blue 22), 1,4-Bis-(2-Hydroxyethyl)amino-5,8-dihydroxy-anthrachinon (Disperse Blue 7) und 1-Methylamino-4-aminopropylamino-anthrachinon (HC Blue 8).

Beispiele für Triphenylmethan-Farbstoffe sind [4-[[4-Diethylamino]-phenyl][4-(ethylamino)-1-naphthalinyl] methylen]-2,5-cyclohexadien-1-yliden]-N-ethyl-ethanamin (Basic Blue 7) und 4',4',4"-Triamino-3-methyltriphenylcarbeniumchlorid (Basic Violet 14, Fuchsin AN).

Die Menge der zugesetzten direktziehenden Farbstoffe beträgt vorzugsweise 0,01 bis 5 Gewichtsprozent, insbesondere 0,1 und 4 Gewichtsprozent.

Die Farbträgermasse kann ausschließlich aus den Verbindungen der Formel (I), gegebenenfalls im Gemisch mit den vorgenannten direktziehenden Farbstoffen, bestehen. Es ist jedoch ebenfalls möglich, daß die Verbindung der Formel (I) in der Farbträgermasse in einem geeigneten kosmetischen Träger vorliegt. In diesem Falle enthält die Farbträgermasse die Verbindung der Formel (I) in einer Menge von etwa 0,001 bis 10 Gewichtsprozent, insbesondere in einer Menge von 0,1 bis 5 Gewichtsprozent.

Das erfindungsgemäße Färbemittel ergibt auf keratinischen Fasem in Gegenwart von Luft blaue Färbungen, die eine sehr gute Stabilität gegenüber kosmetischen Behandlungen, Schweißabsonderungen oder Einwirkungen von Licht und Wetter aufweisen. Weiterhin wird das Haar sehr gleichmäßig und intensiv gefärbt, ohne daß wasserunlösliche Rückstände auf dem Haar verbleiben.

Die Zubereitungsform für die Farbträgermasse sowie für das gebrauchsfertige Färbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion, sowie im Falle der Farbträgermasse ein Pulver oder Granulat. Ihre Zusammensetzung stellt eine Mischung der Farbstoffkomponenten mit den für solche Zubereitungen üblichen Zusätzen dar.

Übliche kosmetische Zusätze sind zum Beispiel Lösungsmittel wie Wasser; niedere aliphatische einwertige oder mehrwertige Alkohole, deren Ester und Ether, beispielsweise Alkanole, insbesondere mit 1 bis 4 Kohlenstoffatomen in der Alkylkette, wie Ethanol, n-Propanol oder Isopropanol, Butanol, Isobutanol; zwei- oder dreiwertige Alkohole, insbesondere solche mit 2 bis 6 Kohlenstoffatomen, beispielsweise Ethylenglycol, Propylenglycol, 1,3-Propandiol, 1,4-Butandiol, 1,5-Pentandiol, 1,6-Hexandiol, 1,2,6-Hexantriol, Glycerin, Diethylenglycol, Dipropylenglycol, Polyalkylenglycole, wie Triethylenglycol, Polyethylenglycol, Tripropylenglycol und Polypropylenglycol; niedere Alkylether von mehrwertigen Alkoholen, wie Ethylenglycol-monomethylether, Ethylenglycol-monoethylether, Ethylenglycol-monopropylether oder Ethylenglycolmonobutylether, Diethylenglycol-monomethylether oder Diethylenglykolmonoethylether, Triethylenglycol-monomethylether oder Triethylenglycolmonoethylether; Ketone und Ketoalkohole, insbesondere solche mit 3 bis 7 Kohlenstoffatomen im Molekül, beispielsweise Aceton, Methylethylketon, Diethylketon, Methylisobutylketon, Methylphenylketon, Cyclopentanon, Cyclohexanon und Diacetonalkohol; Ether wie Dibutylether, Tetra-hydrofuran, Dioxan oder Diisopropylether; Ester wie Ethylformiat, Methylformiat, Methylacetat, Ethylacetat, Propylacetat, Butylacetat, Phenylacetat, Ethylenglycolmonoethyletheracetat oder Essigsäurehydroxyethylester; Amide wie Dimethylformamid, Dimethylacetamid oder N-Methyl-pyrrolidon; sowie Harnstoff, Tetramethylharnstoff und Thiodiglycol; weiterhin Netzmittel oder Emulgatoren aus den Klassen der anionischen, kationischen, amphoteren, nichtionogenen oder zwitterionischen ober-flächenaktiven Substanzen, wie zum Beispiel Fettalkoholsulfate, Alkyl-sulfonate, Alkylbenzolsulfonate, Alkyltrimethylammoniumsalze, Alkylbetaine, $\alpha$-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside; Verdickungsmittel wie höhere Fettalkohole, Stärke, Cellulosederivate, Vaseline, Paraffinöl, Fettsäuren und andere Fettkomponenten in emulgierter Form; wasserlösliche polymere Verdickungsmittel, beispielsweise natürliche Gummen, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol; außerdem Pflegestoffe wie Lanolinderivate, Cholesterin, Pantothensäure, wasserlösliche kationische Polymere, Proteinderivate, Provitamine, Vitamine, Pflanzenextrakte, Zukker und Betain; Hilfsstoffe wie Elektrolyte, Antioxidantien, Fettamide, Sequestrierungsmittel, filmbildende Agentien und Konservierungsmittel.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Zur Herstellung des erfindungsgemäßen Färbemittels versetzt man die vorstehend beschriebene Farbträgermasse mit einem geeigneten Enzym in fester beziehungsweise flüssiger Form. Es ist selbstverständlich auch möglich Lösungen beziehungsweise Suspensionen oder Emulsionen des genannten Enzyms in Wasser oder einem aliphatischen einwertigen oder mehrwertigen Alkohol, beispielsweise Ethanol, Isopropanol oder Butanol, zu verwenden. Ebenfalls ist es möglich, das erfindungsgemäße Färbemittel herzustellen, indem man eine Zubereitung, welche das genannte Enzym sowie alle erforderlichen weiteren kosmetischen Zusatzstoffe enthält, mit einer erfindungsgemäßen

Farbträgermasse, welche ausschließlich die Verbindungen der Formel (I), sowie gegebenenfalls direktziehende Farbstoffe, enthält, vermischt.

Als Enzym kommen Hydrolasen, welche in der Lage sind, eine hydrolytische Spaltung der Indolylglycoside der Formel (I) zu katalysieren, in Betracht.

Besonders bevorzugt ist es hierbei, als Enzym Galactosidasen, insbesondere β-Galactosidase, zu verwenden.

Das erfindungsgemäße Haarfärbemittel hat vorzugsweise einen pH-Wert von 8 bis 11.

Für die Anwendung zur Färbung der Haare trägt man eine für die Behandlung ausreichende Menge, je nach Haarfülle, im allgemeinen etwa 10 bis 60 g, des gebrauchsfertigen Färbemittels auf das Haar auf. Man läßt das Gemisch bei etwa 15 bis 50 °C etwa 10 bis 45 Minuten lang, vorzugsweise 30 bis 40 Minuten lang, auf das Haar einwirken, spült sodann das Haar mit Wasser aus und trocknet es. Gegebenenfalls wird im Anschluß an diese Spülung mit einem Shampoo gewaschen und/oder mit einer schwachen organischen Säure, wie zum Beispiel Zitronensäure, Glycolsäure, Milchsäure, Äpfelsäure, Ascorbinsäure oder Weinsäure, nachgespült. Anschließend wird das Haar getrocknet.

Das erfindungsgemäße Haarfärbemittel führt zu Haarfärbungen mit ausgezeichneten Echtheitseigenschaften, insbesondere was die Licht-, Wasch- und Reibechtheit anbetrifft. Hinsichtlich der färberischen Möglichkeiten bietet das erfindungsgemäße Haarfärbemittel je nach Art und Konzentration der Verbindungen der allgemeinen Formel (I) verschiedene Farbnuancen von hellblau über türkis, blau, blauviolett bis purpur. Bemerkenswert ist hierbei die große Farbintensität und die Farbreinheit der erzielbaren Färbungen. Schließlich ist mit Hilfe des beschriebenen Haarfärbemittels auch eine Anfärbung von ergrautem und chemisch nicht vorgeschädigtem Haar problemlos und mit sehr guter Deckkraft möglich. Die hierbei erhaltenen Färbungen sind, unabhängig von der unterschiedlichen Struktur des Haares, gleichmäßig und sehr gut reproduzierbar.

Das nachfolgende Beispiel sollen den Erfindungsgegenstand näher erläutern, ohne ihn jedoch darauf zu beschränken.

## Beispiele

### Beispiel 1: Haarfärbemittel

| | |
|---|---|
| 0,1 g | 5-Brom-4-chlor-3-indolyl-β-D-galactosid (98%ig; $[\alpha]_D^{20}$ = 62° (c=1 in Dimethylformamid/Wasser 1:1)) |
| 0,3 g | Hydroxyethylcellulose |
| 0,1 g | Dinatriumethylendiaminotetraacetat-hydrat |
| 99,5 g | Phosphatpuffer, 0,1 m (Mischung von Kaliumdihydrogenphosphat und Dikaliumhydrogenphosphat in Wasser; auf pH = 7,8 eingestellt) |
| 100,0 g | |

Die vorstehende Farbträgermasse wird unmittelbar vor Gebrauch mit 1 mg β-Galactosidase (aus E. Coli, 380 U/ mg bei pH = 7,8 und 37° C) zum gebrauchsfertigen Haarfärbemittel vermischt.

Gebleichtes Haar wird sodann 40 Minuten bei einer Temperatur von 40° C mit diesem Haarfärbemittel behandelt. Anschließend wird das Haar mit Wasser gespült, gegebenenfalls mit einem Shampoo gewaschen und erneut mit Wasser gespült, und getrocknet. Es resultiert hellblaue Färbung.

Die erhaltene Färbung übersteht bis zu zehn Haarwäschen ohne merklichen Intensitätsverlust.

1 U (= 1 internationalen Einheit) entspricht der Enzymaktivität, welche die Hydrolyse von 1 µmol Glycosid pro Minute bei dem angegebenem pH-Wert und der angegebenen Temperatur katalysiert.

Alle Prozentangaben stellen, soweit nicht anders angegeben, Gewichtsprozente dar.

## Patentansprüche

1. Mittel zur Färbung von Haaren, **dadurch gekennzeichnet, daß** es unmittelbar vor der Anwendung durch Vermischen (i) einer Farbträgermasse, enthaltend eine Verbindung der allgemeinen Formel (I)

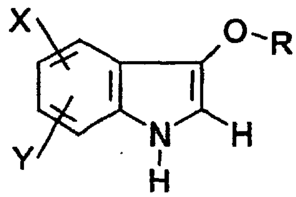

(I) ,

in der R gleich einer DL-, D- oder L-Aldopyranose beziehungsweise DL-, D- oder L-Ketopyranose ist und die Reste X und Y unabhängig voneinander Wasserstoff, ein Halogenatom, eine Hydroxygruppe oder eine Alkoxygruppe mit 1 bis 4 Kohlenstoffatomen bedeuten, mit (ii) einer Hydrolase erhalten wird.

2. Mittel zur Färbung von Haaren nach Anspruch 1, **dadurch gekennzeichnet, daß** die Hydrolase ausgewählt ist aus Galactosidasen.

3. Mittel zur Färbung von Haaren nach Anspruch 1 oder 2, **dadurch gekennzeichnet, daß** als Hydrolase β-Galaktosidase verwendet wird.

4. Mittel nach einem der Ansprüche 1 bis 3, **dadurch gekennzeichnet, daß** die in der Farbträgermasse enthaltene Verbindung der Formel (I) ausgewählt ist aus 5-Brom-4-chlor-3-indolyl-β-D-galactosiden.

5. Mittel nach einem der Ansprüche 1 bis 4, **dadurch gekennzeichnet, daß** die in der Farbträgermasse enthaltene Verbindung der Formel (I) in einem geeigneten kosmetischen Träger in einer Menge von 0,001 bis 10 Gewichtsprozent enthalten ist.

6. Mittel nach einem der Ansprüche 1 bis 5, **dadurch gekennzeichnet, daß** die Farbträgermasse neben der Verbindung der Formel (I) weitere übliche kosmetische Zusätze enthält.

7. Mittel nach einem der Ansprüche 1 bis 5, **dadurch gekennzeichnet, daß** die Farbträgermasse ausschließlich die Verbindung der Formel (I) enthält.

8. Mittel nach einem der Ansprüche 1 bis 7, **dadurch gekennzeichnet, daß** die Farbträgermasse zusätzlich direktziehende Farbstoffe enthält.

9. Mittel nach Anspruch 8, **dadurch gekennzeichnet, daß** der direktziehende Farbstoff ausgewählt ist aus Nitrofarbstoffen, Azofarbstoffen, Anthrachinon-Farbstoffen und Triphenylmethan-Farbstoffen.

10. Mittel nach Anspruch 8 oder 9, **dadurch gekennzeichnet, daß** der direktziehende Farbstoff ausgewählt ist aus Pikraminsäure, 4-(2',3'-Dihydroxypropyl)-amino-3-nitro-trifluor-methylbenzol, 4,N-Ethyl-N-(2'hydroxyethyl)-amino-1-(2'-hydroxyethyl)-amino-2-nitro-benzol, 2-Chlor-6-ethylamino-4-nitrophenol, 1-Hydroxy-2-ß-hydroxyethylamino-4,6-dinitrobenzol, 4-(2'-Hydroxyethyl)-amino-3-nitro-chlorbenzol, 2-Amino-6-chlor-4-nitrophenol und 4-(2'-Hydroxyethyl)-amino-3-nitro-methylbenzol, 1-(2'-Methoxyphenylazo)-2-hydroxy-7-trimethyl-ammonium-naphthalin, 4-(4'-Sulfo-1-phenylazo)-1-(4''-sulfophenyl)-3-carboxy-5-hydroxypyrazolon, 4-Amino-4'-Bis[2''-hydroxyethyl]-amino- azobenzol, 1-(4'-Aminophenylazo)-2-hydroxy-7-trimethylammonium-naphthalin, 1-Methylamino-4-(2'hydroxyethyl)amino-anthrachinon, 1-Amino-4-hydroxy-anthrachinon, 2-Methoxy-1,4-diamino-anthrachinon, 1,4-Diamino-anthrachinon, 1-Amino-4-methylamino-anthrachinon, 1,4-Bis(2',3'-Dihydroxypropyl)-amino-anthrachinon, 1-Methylamino-4-(amino-n-propyltrimethylammonium)-anthrachinon methylsulfat, 1,4-Bis-(2-Hydroxyethyl)amino-5,8-dihydroxy-anthrachinon, 1-Methylamino-4-aminopropylaminoanthrachinon, [4-[[4'-Diethylamino]phenyl][4-(ethylamino)-1-naphthalinyl]-methylen]-2,5-cyclohexadien-1-yliden]-N-ethyl-ethanamin und 4',4',4''-Triamino-3-methyltriphenyl-carbeniumchlorid.

11. Mittel nach einem der Ansprüche 1 bis 10, **dadurch gekennzeichnet, daß** es einen pH-Wert von 8 bis 11 aufweist.

12. Verfahren zum Färben von Haaren, **dadurch gekennzeichnet, daß** ein Haarfärbemittel nach einem der Ansprüche 1 bis 11 auf das Haar aufgetragen wird, bei einer Temperatur von 15 bis 50°C 10 bis 45 Minuten lang einwirken gelassen wird, das Haar anschließend mit Wasser gespült wird, gegebenenfalls schamponiert wird und sodann getrocknet wird.

## Claims

1. Composition for colouring hair, **characterized in that** it is obtained directly prior to use by mixing (i) a colour carrier mass comprising a compound of the general formula (I)

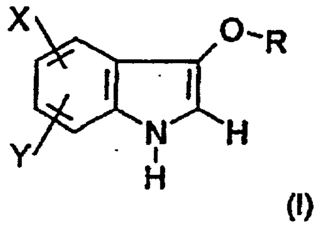

(I) ,

in which R is a DL-, D- or L-aldopyranose or DL-, D- or L-ketopyranose, and the radicals X and Y, independently of one another, are hydrogen, a halogen atom, a hydroxyl group or an alkoxy group having 1 to 4 carbon atoms, with (ii) a hydrolase.

2. Composition for colouring hair according to Claim 1, **characterized in that** the hydrolase is chosen from galactosidases.

3. Composition for colouring hair according to Claim 1 or 2, **characterized in that** the hydrolase used is β-galactosidase.

4. Composition according to one of Claims 1 to 3, **characterized in that** the compound of the formula (I) present in the colour carrier mass is chosen from 5-bromo-4-chloro-3-indolyl-β-D-galactosides.

5. Composition according to one of Claims 1 to 4, **characterized in that** the compound of the formula (I) present in the colour carrier mass is present in a suitable cosmetic carrier in an amount of from 0.001 to 10 per cent by weight.

6. Composition according to one of Claims 1 to 5, **characterized in that** the colour carrier mass comprises further customary cosmetic additives as well as the compound of the formula (I).

7. Composition according to one of Claims 1 to 5, **characterized in that** the colour carrier mass comprises exclusively the compound of the formula (I).

8. Composition according to one of Claims 1 to 7, **characterized in that** the colour carrier mass additionally comprises direct dyes.

9. Composition according to Claim 8, **characterized in that** the direct dye is chosen from nitro dyes, azo dyes, anthraquinone dyes and triphenylmethane dyes.

10. Composition according to Claim 8 or 9, **characterized in that** the direct dye is chosen from picramic acid, 4-(2',3'-dihydroxypropyl)amino-3-nitrotrifluoromethylbenzene, 4,N-ethyl-N-(2'-hydroxyethyl)amino-1-(2'-hydroxyethyl) amino-2-nitrobenzene, 2-chloro-6-ethylamino-4-nitrophenol, 1-hydroxy-2-β-hydroxyethylamino-4,6-dinitrobenzene, 4-(2'-hydroxyethyl)amino-3-nitrochlorobenzene, 2-amino-6-chloro-4-nitrophenol and 4-(2'-hydroxyethyl) amino-3-nitromethylbenzene, 1-(2'-methoxyphenylazo)-2-hydroxy-7-trimethylammonium naphthalene, 4-(4'-sulpho-1-phenylazo)-1-(4"-sulphophenyl)-3-carboxy-5-hydroxypyrazolone, 4-amino-4'-bis[2"-hydroxyethyl]aminoazobenzene, 1-(4'-aminophenylazo)-2-hydroxy-7-trimethylammonium naphthalene, 1-methylamino-4-(2'-hydroxyethyl) aminoanthraquinone, 1-amino-4-hydroxyanthraquinone, 2-methoxy-1,4-diaminoanthraquinone, 1,4-diaminoanthraquinone, 1-amino-4-methylaminoanthraquinone, 1,4-bis(2',3'-dihydroxypropyl)aminoanthraquinone, 1-methylamino-4-(amino-n-propyltrimethylammonium)anthraquinone methylsulphate, 1,4-bis(2-hydroxyethyl) amino-5,8-dihydroxyanthraquinone, 1-methylamino-4-aminopropylaminoanthraquinone, [4-[[4'-diethylamino]phenyl] {4-(ethylamino)-1-naphthalinyl]-methylene]-2,5-cyclohexadien-1-ylidene]-N-ethylethanamine and 4',4',4"-triamino-3-methyltriphenylcarbenium chloride.

11. Composition according to one of Claims 1 to 10, **characterized in that** it has a pH of from 8 to 11.

12. Method of colouring hair, **characterized in that** a hair colorant according to one of Claims 1 to 11 is applied to the hair, left to act at a temperature of from 15 to 50°C for 10 to 45 minutes, the hair is then rinsed with water, optionally shampooed and then dried.

## Revendications

1. Produit pour la teinture des cheveux, **caractérisé en ce qu'**il est obtenu immédiatement avant l'emploi, par mélange de (i) une matière colorante contenant un composé de formule générale (I)

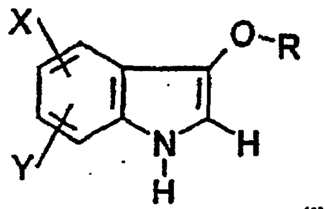

(I) ,

dans laquelle R représente un DL-, D- ou L-aldopyrannose ou un DL-, D- ou L-cétopyrannose et les radicaux X et Y représentent, indépendamment l'un de l'autre, un atome d'hydrogène ou d'halogène, ou un groupe hydroxy ou un groupe alcoxy ayant de 1 à 4 atomes de carbone, avec (ii) une hydrolase.

2. Produit pour la teinture des cheveux selon la revendication 1, **caractérisé en ce que** l'hydrolase est choisie parmi des galactosidases.

3. Produit pour la teinture des cheveux selon la revendication 1 ou 2, **caractérisé en ce qu'**on utilise comme hydrolase la β-galactosidase.

4. Produit selon l'une quelconque des revendications 1 à 3, **caractérisé en ce que** le composé de formule (I) contenu dans la matière colorante est choisi parmi les 5-bromo-4-chloro-3-indolyl-β-D-galactosides.

5. Produit selon l'une quelconque des revendications 1 à 4, **caractérisé en ce que** le composé de formule (I) contenu dans la matière colorante est contenu dans un véhicule cosmétique approprié en une quantité de 0,001 à 10 % en poids.

6. Produit selon l'une quelconque des revendications 1 à 5, **caractérisé en ce que**, outre le composé de formule (I), la matière colorante contient d'autres additifs cosmétiques usuels.

7. Produit selon l'une quelconque des revendications 1 à 5, **caractérisé en ce que** la matière colorante contient exclusivement le composé de formule (I).

8. Produit selon l'une quelconque des revendications 1 à 7, **caractérisé en ce que** la matière colorante contient en outre des colorants directs.

9. Produit selon la revendication 8, **caractérisé en ce que** le colorant direct est choisi parmi les colorants nitrés, les colorants azoïques, les colorants anthraquinoniques et les colorants triphénylméthane.

10. Produit selon la revendication 8 ou 9, **caractérisé en ce que** le colorant direct est choisi parmi l'acide picramique, le 4-(2',3'-dihydroxypropyl)-amino-3-nitrotrifluorométhylbenzène, le 4,N-éthyl-N-(2'-hydroxyéthyl)-amino-1-(2'-hydroxyéthyl)-amino-2-nitrobenzène, le 2-chloro-6-éthylamino-4-nitrophénol, le 1-hydroxy-2-β-hydroxyéthylamino-4,6-dinitrobenzène, le 4-(2'-hydroxyéthyl)-amino-3-nitro-chlorobenzène, le 2-amino-6-chloro-4-nitrophénol et le 4-(2'-hydroxyéthyl)amino-3-nitro-méthylbenzène, le 1-(2'-méthoxyphénylazo)-2-hydroxy-7-triméthyl-ammoniumnaphtalène, la 4-(4'-sulfo-1-phénylazo)-1-(4''-sulfophényl)-3-carboxy-5-hydroxypyrazolone, le 4-amino-4'-bis[2''-

hydroxyéthyl]-amino-azobenzène, le 1-(4'-aminophénylazo) -2-hydroxy-7-triméthylammoniumnaphtalène, la l-méthylamino-4-(2'-hydroxyéthyl)aminoanthraquinone, la 1-amino-4-hydroxy-anthraquinone, la 2-méthoxy-1,4-diamino-anthraquinone, la 1,4-diaminoanthraquinone, la 1-amino-4-méthylamino-anthraquinone, la 1,4-bis(2',3'-dihydroxypropyl)amino-anthraquinone, le méthylsulfate de 1-méthylamino-4-(amino-n-propyltriméthylammonium)-anthraquinone, la 1,4-bis-(2-hydroxyéthyl)amino-5,8-dihydroxy-anthraquinone, la 1-méthylamino-4-aminopropylaminoanthraquinone, la [4-[[4'-diéthylamino]phényl][4-(éthylamino)-1-naphtalinyl]méthylène]-2,5-cyclohexadién-1-ylidène]-N-éthyl-éthanamine et le chlorure de 4',4',4"-triamino-3-méthyltriphényl-carbénium.

**11.** Produit selon l'une quelconque des revendications 1 à 10, **caractérisé en ce qu'**il présente un pH de 8 à 11.

**12.** Procédé pour la teinture des cheveux, **caractérisé en ce qu'**on applique sur les cheveux un produit de teinture pour cheveux selon l'une quelconque des revendications 1 à 11, on laisse agir pendant 10 à 45 minutes à une température de 15 à 50°C, puis on rince les cheveux à l'eau, éventuellement on les shampooine et ensuite on les sèche.